# EUROPEAN PATENT SPECIFICATION

(11) **EP 2 846 736 B1**
(45) Date of publication and mention of the grant of the patent: **28.02.2018**
(21) Application number: 13724090.9
(22) Date of filing: 07.05.2013
(51) Int. Cl.: A61F 2/24

(54) **REDUCED PROFILE VALVE WITH LOCKING ELEMENTS**
VENTIL MIT REDUZIERTEM PROFIL UND VERRIEGELUNGSELEMENTEN
VALVULE À PROFIL RÉDUIT AVEC ÉLÉMENTS DE VERROUILLAGE

(30) Priority: 09.05.2012 US 201261644673 P
(43) Date of publication of application: 18.03.2015
(73) Proprietor: Boston Scientific Scimed, Inc., Maple Grove, MN 55311-1566 (US)
(72) Inventor: SUTTON, Benjamin, T., Scotts Valley, California 95066 (US); GREGG, Peter, W., Santa Cruz, California 95062 (US); PAUL, David, J., Scotts Valley, California 95066 (US); CARROLL, Stanley, A., San Carlos, California 94070 (US)
(74) Representative: Peterreins Schley
(86) International application number: PCT/US2013/039913
(87) International publication number: WO 2013/169748

(56) References cited:
- WO-A1-03/047468
- WO-A1-2010/098857
- WO-A2-2005/062980

## Description

### BACKGROUND OF THE INVENTION

The present invention relates to an apparatus for endovascularly repairing or replacing a heart valve and a method of manufacturing as set forth in the claims. More particularly, the present invention relates to an apparatus for percutaneously repairing or replacing a native heart valve with a replacement valve using an expandable and retrievable anchor.

Heart valve surgery is an often highly invasive operation used to repair or replace a patient's heart valve when there is a narrowing of the native heart valve, commonly referred to as stenosis, or when the native valve leaks or regurgitates. Heart valve surgery is normally an open-heart procedure conducted under general anesthesia. An incision is made through the patient's sternum (sternotomy), and the patient's heart is stopped while blood flow is rerouted through a heart-lung bypass machine.

When replacing the valve, the native valve is excised and replaced with either a biologic or a mechanical valve. Mechanical valves require lifelong anticoagulant medication to prevent blood clot formation, and clicking of the valve often may be heard through the chest. Biologic tissue valves typically do not require such medication. Tissue valves may be obtained from cadavers or may be porcine or bovine, and are commonly attached to synthetic rings that are secured to the patient's heart.

Implantable medical devices can be delivered to a target location within a patient and implanted therein. For example, endoluminal delivery techniques are well known. The delivery system typically includes a sheath and/or a catheter through which the implant is delivered to the target location. The implant is generally deployed from the sheath or catheter at the target location. Some implantable devices are completely self-expanding; they self-expand when released from the sheath or catheter and do not require any further expansion after the self-expanding step. The self-expansion can occur by proximally retracting the sheath or catheter, by pushing the implantable device from the sheath or catheter, or a combination thereof. Some implantable devices, however, are configured and adapted to be actuated during or after the self-expansion step. Exemplary replacement heart valves that can be actuated after a self-expansion step can be found described in US Patent Publication Nos. 2005/0143809 and 2005/0137686. It may be advantageous to lock an expandable medical device in a fully deployed and locked configuration to secure the device in the deployed configuration.

During the delivery process the medical device can be actuated by the delivery system using one or more actuators. For example, an actuator (e.g., in the form of a knob on a handle of the delivery system) may be actuated (e.g., turned) to cause a component of the delivery system to move relative to another component in the delivery system or relative to the implantable device, or both. It is generally desirable to make the delivery process as easy as possible for the physician, reduce the time needed to complete the procedure, and reduce the mechanical complexity of the delivery system. In some delivery procedures, multiple components of the delivery system need to be actuated to deploy the implant. It may be desirable to have a delivery system with a low profile for endoluminal delivery.

WO 2005/062980 describes an apparatus comprising an anchor and a replacement valve. The apparatus may also comprise a lock or a plurality of locks.

### BRIEF SUMMARY OF THE INVENTION

In one aspect of the invention, an apparatus for endovascularly replacing a patient's heart valve comprises at least an anchor having an outer surface and an inner surface. The anchor is expandable from a collapsed delivery configuration to a fully deployed configuration. The anchor is further expandable from the collapsed delivery configuration to an at-rest configuration and from the at-rest configuration to the fully deployed configuration. A first locking element and a second locking element are attached to the inner surface of the anchor. The first locking element is engageable with the second locking element.

At least one of the first locking element and the second locking element has a curved surface. In at least one embodiment, the second locking element comprises a plate with an inner surface and a curved outer surface. In at least one embodiment, the second locking element further comprises a tooth pivotally engaged to the inner surface of the plate. In at least one embodiment, the second locking element further comprises a channel defined by the plate and the tooth, where the first locking element is engageable with the channel during locking and unlocking.

In at least one embodiment, the second locking element is attached to the anchor with an attachment member. In one embodiment, this is a thread-like member. In at least one embodiment, the second locking element has a first plurality of holes near a first end and a second plurality of holes near a second end, where the first plurality of holes and the second plurality of holes are separated by at least a portion of the outer surface. In at least one embodiment, the attachment member is threaded through the first plurality of holes and the second plurality of holes to attach the second locking element to the anchor. In one embodiment, the attachment member crosses the outer surface between the first plurality of holes and the second plurality of holes only once.

At least one method of attaching a locking member to an anchoring element of an apparatus for endovascularly replacing a patient's heart valve is also provided herein. A locking member is placed in contact with an inner surface of the anchoring element, wherein the locking member comprises a plate with a curved outer surface, an inner surface, and a thickness therebetween. A plurality of holes extend through the thickness of the plate, wherein the plurality of holes comprises a first plurality of holes and a second plurality of holes. The first plurality of holes and the second plurality of holes are separated by a portion of the curved outer surface. In at least one embodiment, the curved outer surface contacts the inner surface of the anchoring element. A thread-like attachment member is threaded through the plurality of holes such that the thread-like member only crosses the outer surface between the first plurality of holes and the second plurality of holes once to securely attach the locking member to the anchoring element. In one embodiment, after threading the thread-like member through the plurality of holes, at least one knot is formed with the thread-like member.

### BRIEF DESCRIPTION OF THE SEVERAL VIEWS OF THE DRAWING(S)

FIG. 1A shows an exemplary replacement heart valve in an expanded configuration.
FIG. IB shows the exemplary replacement heart valve in a collapsed configuration.
FIGS. 2A-2B illustrate an exemplary embodiment of a delivery system used to deliver the replacement heart valve shown in FIGS. 2A-2B.
FIG. 3 shows a cross-section of an exemplary prior art replacement heart valve in the collapsed configuration, using prior art buckles.
FIG. 4A shows a perspective view of an exemplary buckle of the present invention.
FIG. 4B shows a perspective view of an exemplary buckle of the present invention.
FIG. 5 shows a schematic cross-section of the exemplary replacement heart valve in the collapsed delivery configuration, with the exemplary buckles of FIGS. 4A-4B.
FIG. 6 shows a cross-sectional view of the exemplary replacement heart valve in the collapsed delivery configuration, with buckles similar to the exemplary buckles of FIGS. 4A-4B.
FIGS. 7A-7B show a schematic cross-section of the exemplary replacement heart valve in the collapsed delivery configuration, with the exemplary posts of the present invention.
FIG. 8 shows a cross-sectional view of the exemplary replacement heart valve in the expanded delivery configuration with the exemplary buckle of FIGS. 4A- 4B.
FIG. 9 shows the exemplary buckle of FIGS. 4A-4B as attached to the expandable anchor of the exemplary replacement valve of the present invention.
FIGS. 10A-10H show schematic views of the procedure for attaching the exemplary buckle to the exemplary anchor shown in FIG. 9.

### DETAILED DESCRIPTION OF THE INVENTION

While this invention may be embodied in many different forms, there are described in detail herein specific preferred embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

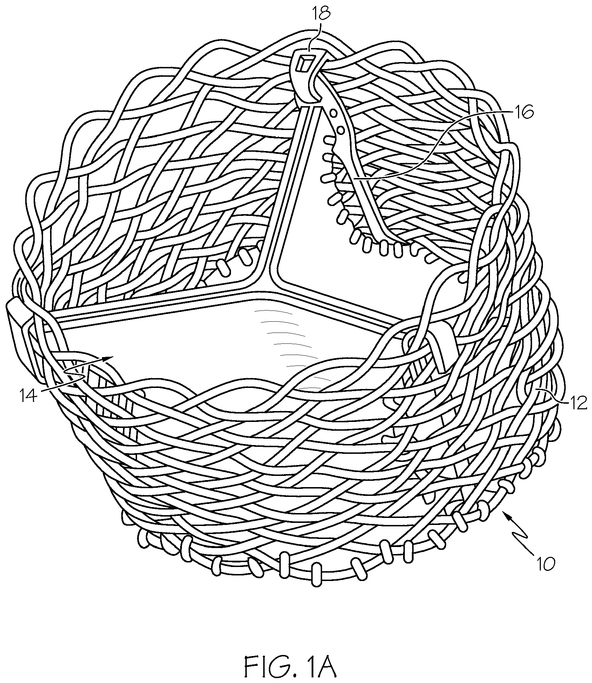

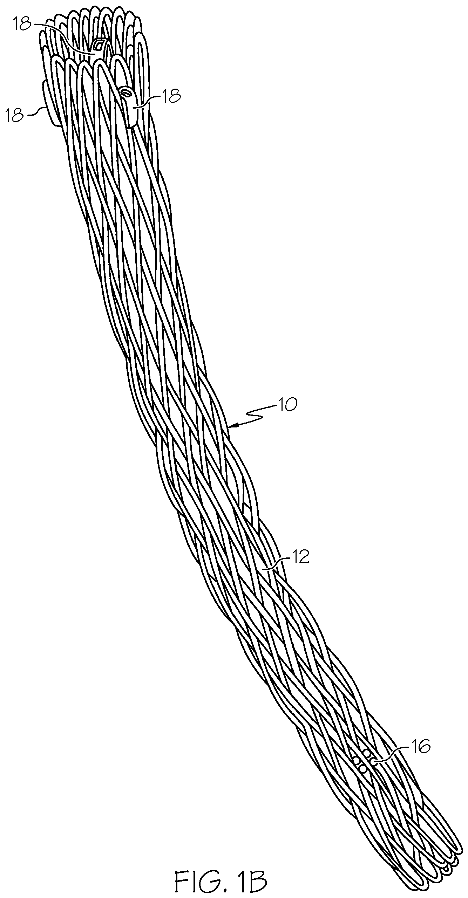

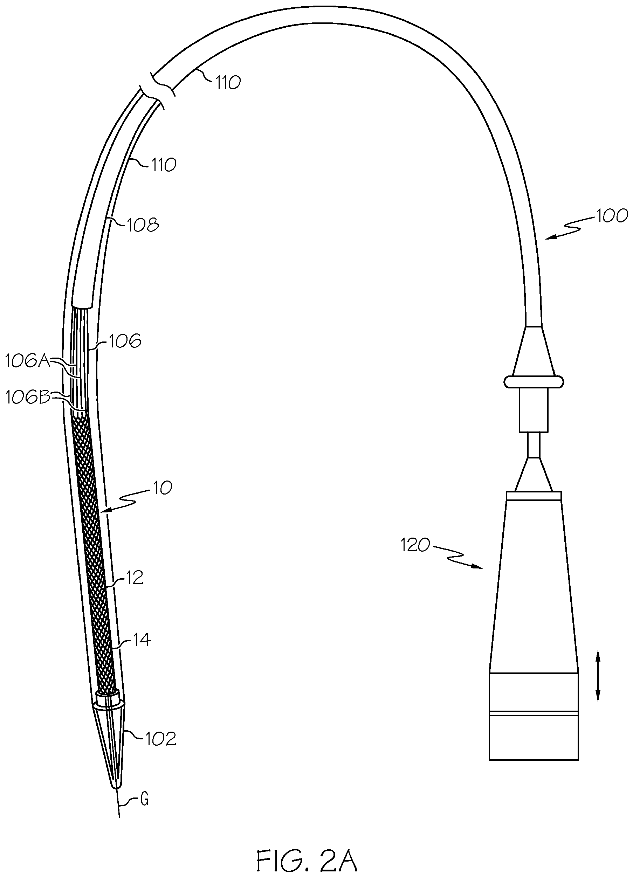

Replacement heart valves shown in FIGS. 1A-2B are known in the art. FIGS. 1A-1B show an exemplary replacement heart valve 10 comprising an expandable anchoring element 12 (also referred to herein as "anchor"), replacement valve leaflets 14 (not shown in FIG. IB for clarity), a plurality of first locking members 16 (also referred to herein as "posts") and a plurality of second locking members 18 (also referred to herein as "buckles"). FIG. 1A shows anchoring element 12 (or "anchor") in a fully deployed configuration in which the anchoring element 12 is locked and maintained in the fully deployed configuration by the locking interaction between first locking members 16 ("posts") and second locking members 18 ("buckles"). FIG. IB shows the anchoring element 12 in a collapsed delivery configuration as the replacement heart valve is delivered within a delivery system (shown in FIGS. 2A-2B) to a target location within the subject.

In at least one embodiment, the anchoring element 12 is expandable from the collapsed delivery configuration to the at-rest (or partially deployed) configuration, and further expandable from the at-rest configuration to the fully deployed configuration. In the embodiments shown, the anchoring element 12 comprises a braided material and is formed of one or more strands of material. In at least one embodiment, the material is a shape memory material. In at least one embodiment, the anchoring element 12 is heat set in the at-rest configuration, such that when the anchoring element 12 is deployed from the sheath of the delivery system, the anchoring element 12 will begin to naturally begin to shorten and self-expand from the collapsed delivery configuration to the at-rest configuration.

In at least the embodiment shown, the valve leaflets 14 are attached to the posts 16 at the valve's three commissures. Posts 16 therefore support the valve leaflets 14 within the anchoring element 12. The posts 16 and buckles 18 (or other suitable first locking members and second locking members) are both coupled to the anchoring element 12. Posts 16 are moveably coupled to the anchoring element 12 at a location distal to the proximal end of the anchoring element 12. Buckles 18 are affixed to the anchoring element (but also may be moveably coupled to the anchoring element like the posts) at a proximal region of the anchoring element 12.

As shown in FIGS. 1A-1B, each post 16 is associated with one of the buckles 18. As shown in FIGS. 1A-1B, the replacement heart valve 10 has three posts 16 and three buckles 18. As shown in FIG. 1A, the replacement heart valve 10 has three replacement valve leaflets 14. In at least one embodiment, the replacement heart valve 10 has as many first locking members 16 as second locking members 18. In at least one embodiment, the replacement heart valve has as many replacement valve leaflets 14 as first locking members 16. In at least one embodiment, the replacement heart valve has as many replacement valve leaflets 14 as second locking members 18.

In at least one embodiment, each post 16 has a locking element that is configured to lock with a corresponding locking element of the associated buckle 18. When the anchoring element 12 is in the collapsed configuration as shown in FIG. IB, each locking element of posts 16 is located proximally relative to the locking element of the buckle 18 to which is it to adapted to be locked.

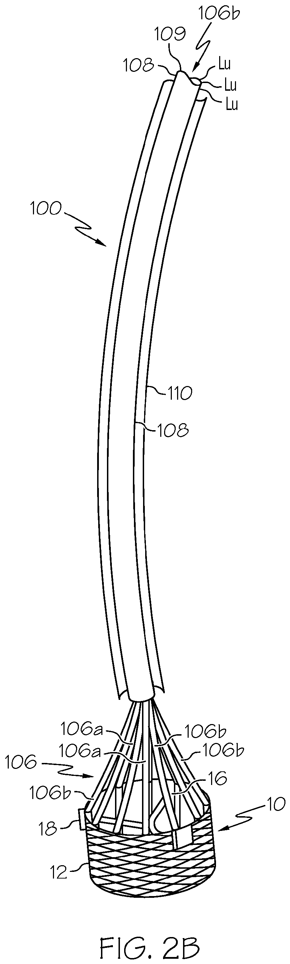

FIGS. 2A and 2B illustrate an exemplary embodiment of a delivery system 100 and components thereof which can be used to deliver the replacement heart valve 10. Delivery system 100 includes handle 120, sheath 110, catheter 108 disposed with sheath 110, and actuation elements 106A and 106B which are reversibly coupled to replacement heart valve 10. Delivery system 100 also includes guidewire G and nose cone 102. In some embodiments, catheter 108 has central lumen 109 and a plurality of circumferentially disposed lumens Lu. In FIG. 2A, the replacement heart valve 10 is in a collapsed delivery configuration (also shown in FIG. IB) within the sheath 110 of the delivery system 100. In FIG. 2B, the exemplary delivery system 100 is reversibly coupled to the replacement heart valve, and the replacement heart valve is in a deployed and locked configuration. More particularly, the plurality of actuation elements 106A are shown reversibly coupled to a proximal region of anchoring element 12 via a reversible coupling mechanism. Actuation elements 106B are reversibly coupled to a region of the replacement heart valve distal to the proximal end of the anchoring element via a reversible coupling mechanism.

In the embodiments shown, the anchoring element comprises a braided material, such as nitinol, and is formed of one or more strands of material. In one embodiment the anchoring element 12 is formed of a shape memory material and is heat set in a self-expanded configuration, such that when the anchoring element is deployed from the sheath of the delivery system, the braid will begin to naturally begin to shorten and expand from the collapsed delivery configuration to the at-rest or partially deployed configuration. This is described in more detail in U.S. Patent Publication Nos. 2005/0137686 and 2005/0143809. Once the anchoring element 12 has expanded to the at-rest (or partially deployed) configuration, at least one of the actuators 106A and 106B is actuated via an actuator on a handle disposed external to the patient. The actuators are described in more detail at least in U.S. Patent Publication Nos. 2005/0137686 and 2005/0143809. Actuators 106B can be actuated in the proximal direction relative to the actuation elements 106A, which applies a proximally directed force to a distal region of the anchoring element.
Actuators 106A can, alternatively or in addition to the proximally directed force, be actuated in a distal direction to apply a distally directed force on a proximal region of the anchoring element 12. The axially directed forces actively foreshorten the anchoring element, moving the posts 16 closer to the buckles 18 until the posts 16 and buckles 18 lock together to lock the anchoring element 12 in a fully deployed and locked configuration. The anchoring element 12 in the locked configuration is therefore shorter than it is in the partially-deployed configuration.

The above provides a general description of an exemplary replacement heart valve. Additional details of this and similar embodiments of replacement heart valves, including details of various aspects of such valves and delivery, deployment, locking, repositioning, and release processes that may be incorporated into this and other embodiments can be found at least in U.S. Patent Nos. 7329279, 7381219, 7445631, 7748389, 7780725, 7824442, 7824443; U.S. Patent Publication Nos. 2005/0112355, 2005/0137686, 2005/0137687, 2005/0137688, 2005/0137689, 2005/0137691, 2005/0137692, 2005/0137694, 2005/0137695, 2005/0137696, 2005/0137697, 2005/0137701, 2005/0143809, 2006/0058872, 2006/0173524, 2006/0253191, 2007/0010876, 2007/0024452, 2007/0112355, 2007/0118214, 2007/0162107, 2007/0203503, 2008/0125859, 2008/0234814, 2009/0076598, 2009/0054969, 2009/0264997, 2010/0121434, 2010/0280495; and WO Publication Nos. 2005/062980, 2005/065585, 2006/009690, 2007/053243, 2007/058847.

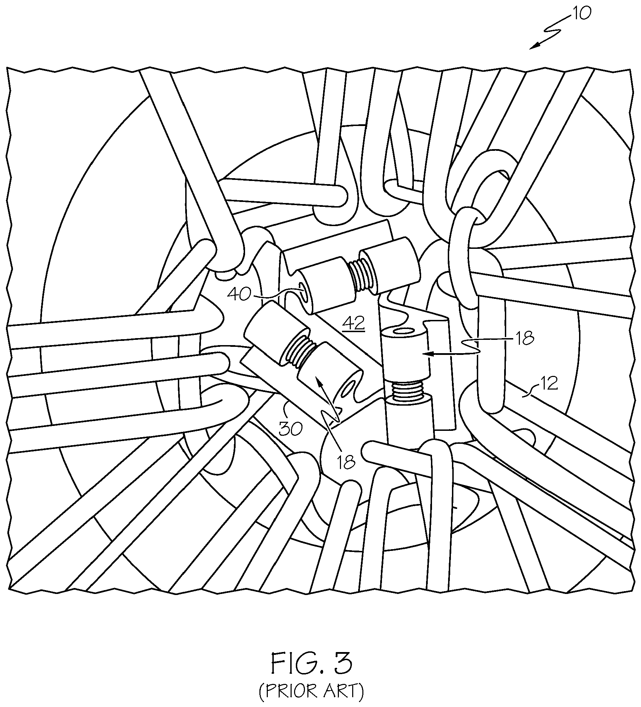

FIG. 3 shows a cross-section of the replacement heart valve 10, with the anchoring element 12 in the collapsed delivery configuration shown in FIGS. 1A and 2A. Importantly, as shown in this figure, the prior art buckles 18 have a flat outer surface 30. As shown, when in the collapsed delivery configuration, the buckles 18 are undesirably impacted in this configuration such that, in some cases, the buckles 18 abut one another. As shown, the buckles 18 are not evenly spaced apart. As shown, ends 40 of at least one buckle 18 abut the inner surface 42 of an adjacent buckle 18. The uneven spacing and contact between the buckles can result in distortions in the delivery profile of the expandable anchoring element 12 and can hinder the ability of the catheter 108 to properly function. In at least one embodiment, the contact of the buckles 18 may inhibit a guidewire or other structure from being easily inserted within the device 10. In at least one embodiment, the delivery profile of the device may be unpredictable due to the uneven spacing and contact between the buckles. Similarly, the prior art posts (not shown) have a flat outer surface and when in the collapsed delivery configuration, the buckles 18 are undesirably impacted in this configuration, resulting in distortions in the delivery profile and inhibiting the ability of a guidewire or other structure from being easily inserted within the device 10.

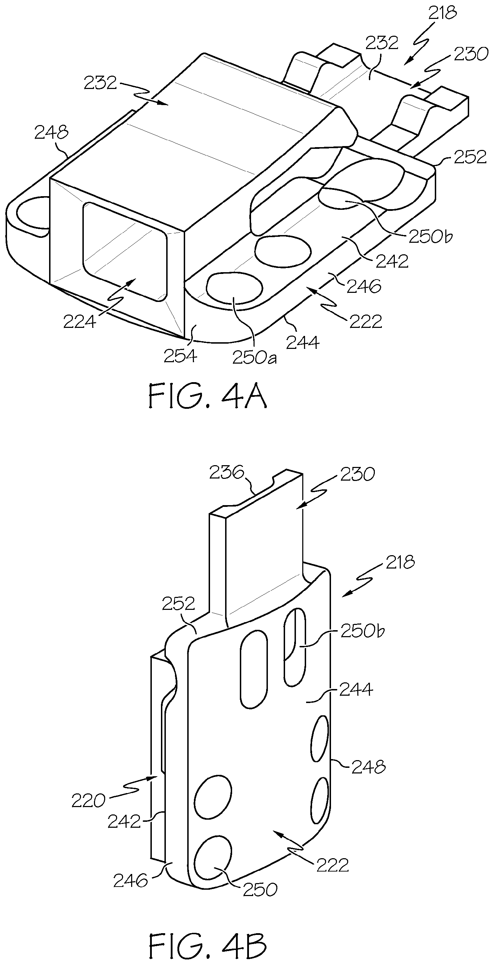

The inventive buckle and/or post described herein correct the issues described above with respect to prior art configurations. FIGS. 4A-4B show an improved buckle 218 of the present invention. Buckle 218 comprises a tooth 220 joined to a plate 222, wherein the tooth 220 and the plate 222 define a channel 224. In at least one embodiment, the tooth 220 is pivotally engaged to the plate 222. In at least one embodiment, the tooth 220 is mounted on the plate near a first end 254 of the plate. A post (not shown) can engage and/or disengage with the tooth 220 and the channel 224 to lock the anchoring element (not shown) into the fully deployed configuration, as discussed above. An example of this engagement and/or disengagement is shown in US Patent Publication No. 2010/0280495. In at least one embodiment, a tab extension 230 extends outwardly from the plate at a second end 252 of the plate 222 and parallel to the tooth 220. The tab extension 230 has a groove 232 that is substantially aligned with the channel 224. The groove 232 allows for the actuation members to engage with the buckle to lock or unlock the anchoring element 212 in a fully deployed configuration.

In at least the embodiments shown, the plate 222 has an inner surface 242, an outer surface 244, and a thickness therebetween. The tooth 220 is attached to the inner surface 242 of plate 22 and can pivot relative thereto. The plate 222 has a first side 246 and a second side 248. As shown most clearly in FIG. 4B, the outer surface 244 is curved from side 246 to side 248. In at least the embodiment shown, this is a single convex curve, but in other embodiments, the curved surface may be concavely curved or may include multiple curved portions. In some embodiments the inner surface 242 is curved rather than the outer surface 244. In at least one embodiment, both the inner surface 242 and the outer surface 244 are curved.

In at least one embodiment, the outer surface 244 has a similar radius of curvature as the anchoring element 212 in the fully deployed configuration. In one embodiment, the outer surface 244 has a similar radius of curvature as the anchoring element 212 in the collapsed delivery configuration.

The radius of curvature of the outer surface 244 is between the radius of curvature of the anchoring element 212 in the collapsed delivery configuration and the radius of curvature of the anchoring element 212 in the fully deployed configuration. In at least one embodiment, the radius of curvature of the outer surface of the buckle is a weighted average of the radius of curvature of the anchoring element 212 in the collapsed delivery configuration and the radius of curvature of the anchoring element 212 in the fully deployed configuration. In at least one embodiment, the weighted average is determined by the following equation: rBuckle= x (rDeployed) + (1- x)(rCollapsed), where x is a weighted factor. In one embodiment, the weighted factor is between about 0.10 and 0.35. In one embodiment, the weighted factor is between about 0.25 and 0.26.

In at least one embodiment, the curved outer surface 244 of the buckle has an arc length between about 10% and about 30% of the circumference of the inner surface of the anchoring element 212 in the collapsed configuration. Preferably, the arc length is between about 20% and about 25%. In at least one embodiment, the curved outer surface 244 of the buckle has an arc length between about 1% and about 10% of the circumference of the inner surface of the anchoring element 212 in the deployed configuration. Preferably, the arc length is between about 3% and about 7%.

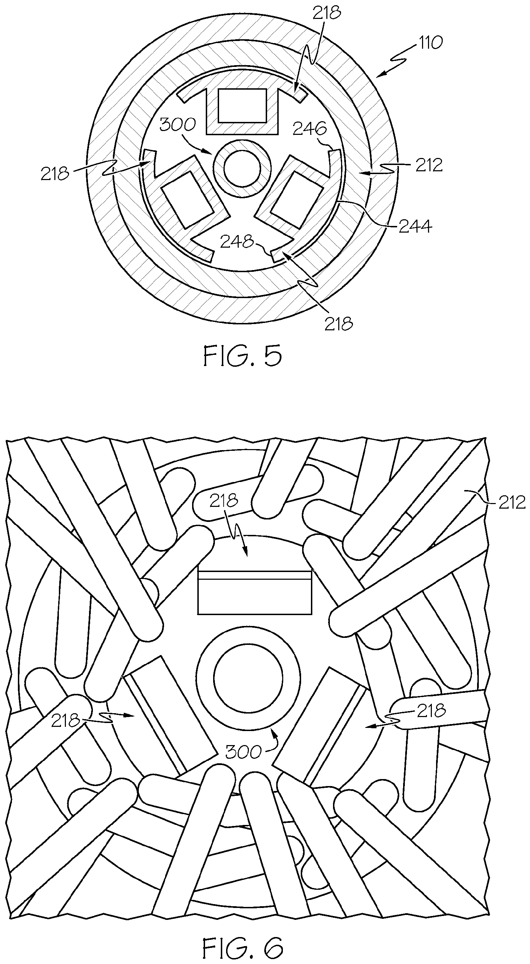

The curved surface, as shown at least in FIGS. 5 and 6, allows the inventive buckles 218 to be spaced apart at least substantially equidistantly in at least the collapsed configuration. Desirably, the buckles 218 are spaced apart equidistantly in each of the collapsed configuration, at-rest configuration, and fully deployed configuration. "Substantially equidistantly" is defined as a difference of between about 0% and about 5% between the spacings between adjacent buckles 216 in one of the configurations. Additionally, the inventive buckle 218 provides a more robust buckle- anchor attachment for adequate in vivo valve durability.

In at least one embodiment, shown in FIG. 5, the tooth 220 does not contact the guidewire assembly 300. In other embodiments, the tooth 220 tangentially contacts the guidewire assembly 300. In some embodiments where the tooth 220 or the inner surface 242 is curved, a portion of the tooth 220 or the inner surface 242 contacts the guidewire assembly 300.

Although the above describes the buckle as having an outer surface with a single curve, the buckle may have an outer surface with multiple curves, or an outer surface that is beveled, or an outer surface with a contour that matches the contours inner surface of the stent.

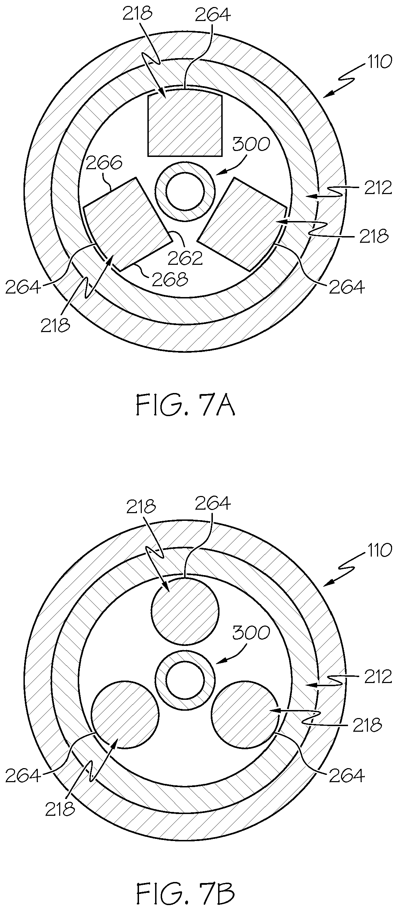

As discussed previously above, the posts may also have a curved surface. As shown in FIG. 7A, the post 216 has an inner surface 262, an outer surface 264, and a thickness therebetween. The tooth 220 is attached to the inner surface 242 of plate 22 and can pivot relative thereto. The post 216 has a proximal end (not shown) and a distal end (not shown), and a first side 266 and a second side 268. The outer surface 244 is curved from side 266 to side 268. In at least the embodiment shown, this is a single convex curve, but in other embodiments, the curved surface may be concavely curved or may include multiple curved portions. In some embodiments the inner surface 262 is curved rather than the outer surface 264. In at least one embodiment, both the inner surface 262 and the outer surface 264 are curved. In at least one embodiment, where the outer surface of the post is curved, the inner surface of the buckle is similarly curved so that the outer surface of the post engages appropriately with the buckle. In one embodiment shown in FIG. 7B, the post has a circular cross-section, rather than just one or two curved surfaces 262, 264. In at least one embodiment, at least the groove 232 of the tooth 230 of the associated buckle is also curved similarly to the outer surface of the post.

In at least one embodiment, the outer surface 264 has a radius of curvature similar to the anchoring element 212 in the fully deployed configuration. In one embodiment, the outer surface 264 has a radius of curvature similar to the anchoring element 212 in the collapsed delivery configuration. In at least one embodiment, the outer surface 264 has a radius of curvature similar to the outer surface 244 of the buckle 218. In at least one embodiment, the sides 266, 268 contact the anchoring element 212 in the collapsed delivery configuration, as shown in FIG. 5. In other embodiments, the side 266, 268 of contact the anchoring element 212 in the collapsed delivery configuration.

The curved outer surface 264 of the posts allows the inventive posts 216 to be spaced apart equidistantly in at least the collapsed configuration. Desirably, the posts 216 are spaced apart substantially equidistantly in each of the collapsed configuration, at-rest configuration, and fully deployed configuration. "Substantially equidistantly" is defined as a difference between about 0% and 5% between the spacings between adjacent posts 216 in one of the configurations. In at least one embodiment, shown in FIG. 7, the posts 216 do not contact the guidewire assembly 300. In other embodiments, the inner surface 262 tangentially contacts the guidewire assembly 300. In some embodiments where the inner surface 262 is curved, a portion of the curved inner surface 262 contacts the guidewire assembly 300.

In at least one embodiment, the curved outer surface 264 of the post has an arc length between about 3% and about 30% of the circumference of the inner surface of the anchoring element 212 in the collapsed configuration. Preferably, the arc length is between about 5% and about 15%. In at least one embodiment, the curved outer surface 264 of the post has an arc length between about 1% and about 5% of the circumference of the inner surface of the anchoring element 212 in the deployed configuration. Preferably, the arc length is between about 2% and about 3%.

Referring back to FIGS. 4A and 4B, in at least the embodiments shown, a plurality of holes 250 extend through the entire thickness between the inner surface 242 and the outer surface 244. Holes 250 may include round throughholes 250a, slotted holes 250b, angled holes (not shown), and other suitable holes. In the embodiments shown in FIGS. 4A and 4B, the buckle 218 has two sets of two round throughholes 250a and two slotted holes 250b.

As shown in FIG. 4A, each slotted hole 250b is aligned with one set of throughholes 250a near each end 246, 248 of the plate. The slotted holes 250b are near the second end 252 of the plate 222 while at least one of the throughholes 250a is near the first end 254 of the plate. As shown in FIG. 4B, the slotted holes 250b are centered on the plate and aligned near the top of the plate, while the two sets of throughholes 250a are located at each end of the plate and aligned near the bottom of the plate. As will be discussed below, these holes 250 are used to attach the buckle 218 to the anchoring element 212.

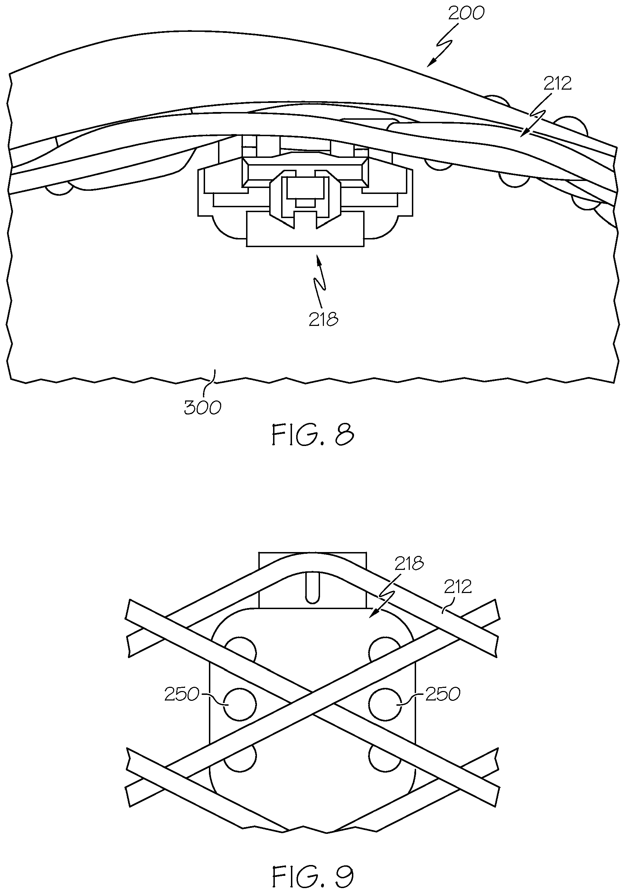

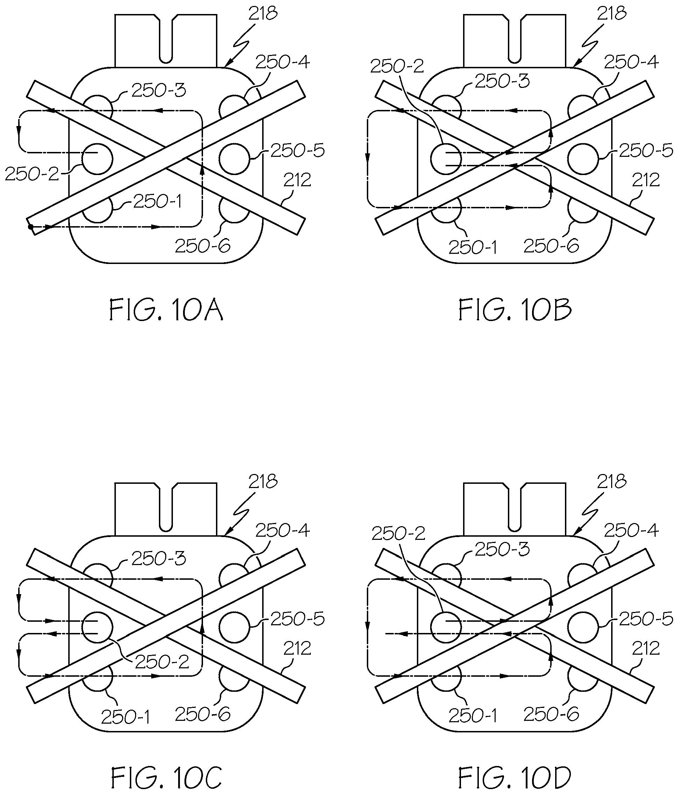

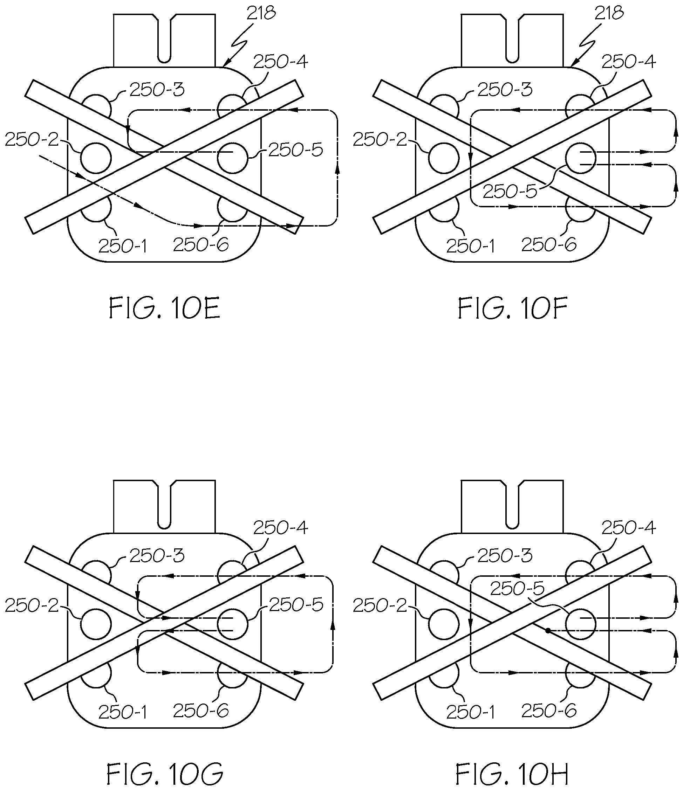

As shown in FIGS. 8 and 9, The buckle is attached to the anchoring element 212 at a particular location of the 212 anchoring element. Where the anchoring element 212 is a braided anchoring element, the center of the buckle 218 is placed at an intersection of filaments of the braided anchoring element, as shown in FIG. 9. The buckle 218 is then attached to the anchoring element 212 with an attachment member 270 (such as a thread-like member, suture, or other like member) using the exemplary method shown in FIGS. 10A-10H. Attachment member 270 may a wire, thread, string, or suture formed from a material selected from the group consisting of metals and polymers. In at least one embodiment, the attachment member is coated with a coating, including, but not limited to, therapeutic coatings. Other methods of attaching the buckle 218 to the anchoring element 212 are contemplated by this invention. The method below can also be used with buckles that do not have a curved surface.

In at least one embodiment the buckle is attached to the anchoring element 212 by threading the attachment member 270 through the plurality of holes 250. In one embodiment, the step of threading the attachment member 270 through the plurality of holes 250 comprises:
(i) inserting a thread-like member into a first hole from the curved outer surface of the plate;
(ii) inserting the thread-like member into a third hole from the inner surface of the plate;
(iii) inserting the thread-like member into a second hole from the curved outer surface of the plate;
(iv) inserting the thread-like member into the third hole from the inner surface of the plate;
(v) inserting the thread-like member into the first hole from the curved outer surface of the plate; and
(vi) inserting the thread-like member into the second hole from the inner surface of the plate.

In some embodiments, steps (i)-(vi) are repeated. In one embodiment, the step of threading the thread-like member through the plurality of holes further comprises:
(vii) inserting the thread-like member into the sixth hole from the curved outer surface of the plate;
(viii) inserting the thread-like member into the fourth hole from the inner surface of the plate;
(ix) inserting the thread-like member into the fifth hole from the curved outer surface of the plate;
(x) inserting the thread-like member into the fourth hole from the inner surface of the plate;
(xi) inserting the thread-like member into the sixth hole from the curved outer surface of the plate; and
(xii) inserting the thread-like member into the fifth hole from the inner surface of the plate.

In some embodiments, steps (vii)-(xii) are also repeated.

As shown in FIGS. 7A-7H, the buckle 218 has three throughholes 250 near one end 246 and three throughholes 250 near the other end 248. (Although the method is described using throughholes, in some embodiments, the method may be used with other hole configurations.) For ease of illustration, the throughholes are individually labeled clockwise from the lower lefthand corner as 250-1 through 250-6. As shown in FIG. 7A, from the outer surface 244 of the buckle 218, the attachment member 270 enters hole 250-1 and then passes through hole 250-3 to form a first stitch. For each "stitch" (which results from the attachment member 270 exiting one hole and entering another hole), the attachment member 270 may go between the inner surface of the buckle and the tooth and loop around to the next hole (as shown with dashed lines in FIG. 7A), or the stitch may be formed using the shortest distance between the holes (similar to a backstitch used in sewing).

In at least one embodiment, the attachment member 270 goes over at least one filament of the braided anchoring element 212. The attachment member 270 then enters hole 250-2 from the outer surface 244 to form a second stitch. As shown in FIG. 7B, the attachment member 270 exits hole 250-2 and passes through hole 250-3. The attachment member goes over at least two filaments of the braided anchoring element 212, and enters hole 250-1 again. The attachment member 270 then passes through hole 250-2. As shown in FIG. *1C*, the attachment member 270 exits hole 2502, goes over at least one filament of the braided anchoring element 212, and enters hole 250-1. The attachment member then passes through hole 250-3 again, goes over at least one filament of the braided anchoring element, and enters hole 250-2 again. As shown in FIG. 7D, after exiting hole 250-2, the attachment member 270 passes through hole 250-3 goes over at least two filaments of the braided anchoring element 212, and enters hole 250-1 again. The attachment member 270 then passes through hole 250-2. As shown in FIG. 7E, the attachment member 270 goes over at least one filament of the braided anchoring element 212, and enters hole 250-6 on the opposite end of the buckle 218. The attachment member 270 then passes through hole 250-4. The attachment member 270 then goes over at least one filament of the braided anchoring element 212, and enters hole 250-5 from the outer surface 244. As shown in FIG. 7F, the attachment member 270 then passes through hole 250-4, wraps around two filaments of the braided anchoring element 212 at the intersection, and enters hole 250-6 again. The attachment member 270 then passes through hole 250-5. As shown in FIG. 7G, the attachment member 270 exits hole 250-5, goes over at least one filament, and enters hole 250-6.

The attachment member then passes through hole 250-4 again, goes over at least one filament, and enters hole 250-5 again. As shown in FIG. 7H, the attachment member 270 passes through hole 250-4, goes over at least two filaments, and enters hole 250-6 again. The attachment member then finally passes through hole 250-5.

Although the above is described with respect to a particular hole arrangement of six holes, the method can be applied to buckles with more or less holes. For instance, the method can comprise:
(i) inserting an attachment member into a first hole from the curved outer surface of the plate;
(ii) inserting the attachment member into a second hole from the inner surface of the plate;
(iii) inserting the attachment member from the curved outer surface of the plate into one hole between the first hole and the second hole;
(iv) inserting the attachment member into the second hole from the inner surface of the plate;
(v) inserting the attachment member into the first hole from the curved outer surface of the plate; and
(vi) inserting the attachment member from the inner surface of the plate into one hole between the first hole and the second hole.

In some embodiments, steps (i)-(vi) are repeated.

In at least one embodiment, the step of threading the attachment member through the plurality of holes further comprises:
(vii) inserting the attachment member into a third hole from the curved outer surface of the plate, wherein the third hole is on an opposite side of the tooth from the first hole;
(viii) inserting the attachment member into a fourth hole from the inner surface of the plate, wherein the third hole is on an opposite side of the tooth from the first hole;
(ix) inserting the attachment member from the curved outer surface of the plate into one hole between the third hole and the fourth hole;
(x) inserting the attachment member into the fourth hole from the inner surface of the plate;
(xi) inserting the attachment member into the third hole from the curved outer surface of the plate; and
(xii) inserting the attachment member from the inner surface of the plate into one hole between the third hole and the fourth hole.

In some embodiments, steps (vii)-(xii) are also repeated.

To secure the attachment member 270, the ends of the attachment member are tied with one slip knot followed by a square knot. Preferably, the knot is ended towards the bottom 254 of the buckle 218. In some embodiments, a heat treatment may be applied to at least a portion of attachment member 270, which may or may not include the knot. In some embodiments, a coating may be applied to at least a portion of attachment member 270, which may or may not include the knot.

While the figures shown herein show only a curved outer surface of the plate, in at least one embodiment the inner surface of the plate is also curved. In at least one embodiment, the radius of curvature of the outer surface is the same as the radius of curvature of the inner surface. In one embodiment, the tooth has a first surface and a second surface parallel to the first surface, the first surface is adjacent to the inner surface of the plate. In at least one embodiment, the second surface of the tooth also has a curved surface with a radius of curvature. In one embodiment, the radius of curvature of the second surface of the tooth is the same as the radius of curvature of the outer surface of the tooth. In one embodiment, the radius of curvature of the second surface of the tooth is the same as the radius of curvature of the outer surface of the tooth.

## Claims

1. An apparatus for endovascularly replacing a patient's heart valve comprising:
an anchor (12, 212) having an outer surface and an inner surface, the anchor (12, 212) expandable from a collapsed delivery configuration to a fully deployed configuration; and
a plurality of first locking elements (16, 216) and a plurality of second locking elements (18, 218) attached to the inner surface of the anchor (12, 212), each first locking element (16, 216) engageable with one second locking element (18, 218), wherein one of the plurality of first locking elements (16, 216) and the plurality of second locking elements (18, 218) have curved outer surfaces (244, 264), wherein each curved outer surface abuts the inner surface of the anchor (12, 212) and each outer surface is curved between a first side and a second side,
**characterized in that** the radius of curvature of the outer surface (244) is between the radius of curvature of the anchor (12, 212) in the collapsed delivery configuration and the radius of curvature of the anchor (12, 212) in the fully deployed configuration.

2. The apparatus of claim 1, wherein in at least the collapsed delivery configuration, the locking elements (16, 216; 18, 218) with curved outer surfaces (244, 264) are spaced substantially equidistantly apart.

3. The apparatus of claim 2, wherein the radius of curvature of the second locking elements (18, 218) is determined by the radius of curvature of the anchor (12, 212) in the fully deployed configuration, the radius of curvature of the anchor (12, 212) in the collapsed delivery configuration, and a weighted factor, in particular wherein the weighted factor is between about 0.10 and 0.35.

4. The apparatus of claim 1, wherein in the collapsed delivery configuration, the curved outer surface (244, 264) has an arc length between about 3% and about 30% of a circumference of the inner surface of the anchor (12, 212).

5. The apparatus of claim 1, wherein the second locking element (18, 218) is attached to the anchor (12, 212) with an attachment member (270).

6. The apparatus of claim 5, wherein the second locking element (18, 218) has a first plurality of holes near a first end and a second plurality of holes near a second end, the first plurality of holes and the second plurality of holes separated by a portion of the outer surface (244, 264), wherein the attachment member (270) is threaded through the first plurality of holes and the second plurality of holes to attach the second locking element (18, 218) to the anchor (12, 212), in particular wherein the attachment member (270) crosses the outer surface (244, 264) between the first plurality of holes and the second plurality of holes only once.

7. The apparatus of claim 1, wherein, the anchor (12, 212) is expandable from the collapsed delivery configuration to an at-rest configuration and from the at-rest configuration to the fully deployed configuration.

8. The apparatus of claim 1, wherein both the plurality of first locking elements (16, 216) and the plurality of second locking elements (18, 218) have curved outer surfaces (244, 264).

9. The apparatus of claim 1, wherein the second locking element (18, 218) comprises:
a plate (222) with an inner surface (242) and a curved outer surface (244); and
a tooth pivotally engaged to the inner surface (242) of the plate (222).

10. The apparatus of claim 9, wherein in the fully deployed configuration, the anchor (12, 212) has a radius of curvature, wherein the curved outer surface (244) of the second locking element (18, 218) has a radius of curvature, wherein the radius of curvature of the anchor (12, 212) is substantially equal to the radius of curvature of the curved outer surface (244).

11. A method of attaching a locking member (18, 218) to an anchoring element (12, 212) of an apparatus for endovascularly replacing a patient's heart valve, the method comprising:
placing a locking member (18, 218) in contact with an inner surface of the anchoring element (12, 212), wherein the locking member (18, 218) comprises a plate (222) with a curved outer surface (244), an inner surface (242), and a thickness therebetween with a plurality of holes (250) extending through the thickness of the plate (222), wherein the plurality of holes (250) comprises a first plurality of holes and a second plurality of holes, wherein the first plurality of holes and the second plurality of holes are separated by a portion of the curved outer surface (244); wherein the curved outer surface (244) contacts the inner surface of the anchoring element (12, 212); and
threading a thread-like member (270) through the plurality of holes (250) such that the thread-like member (270) only crosses the outer surface (244) between the first plurality of holes and the second plurality of holes once to securely attach the locking member (18, 218) to the anchoring element (12,212),
**characterized in that** the radius of curvature of the outer surface (244) is between the radius of curvature of the anchor (12, 212) in the collapsed delivery configuration and the radius of curvature of the anchor (12, 212) in the fully deployed configuration.

12. The method of claim 11, wherein the first plurality of holes comprises at least a first hole, a second hole, and a third hole; and the second plurality of holes comprises at least a fourth hole, a fifth hole and a sixth hole.

13. The method of claim 12, wherein the step of threading the thread-like member (270) through the plurality of holes comprises:
(a) inserting a thread-like member (270) into the first hole from the curved outer surface (244) of the plate (222);
(b) inserting the thread-like member (270) into the third hole from the inner surface (242) of the plate (222);
(c) inserting the thread-like member (270) into the second hole from the curved outer surface (244) of the plate (222);
(d) inserting the thread-like member (270) into the third hole from the inner surface (242) of the plate (222);
(e) inserting the thread-like member (270) into the first hole from the curved outer surface (244) of the plate (222); and
(f) inserting the thread-like member (270) into the second hole from the inner surface (242) of the plate (222), and optionally
(g) repeating steps (a)-(f).

14. The method of claim 13, further comprising:
(h) inserting the thread-like member (270) into the sixth hole from the curved outer surface (244) of the plate (222);
(i) inserting the thread-like member (270) into the fourth hole from the inner surface (242) of the plate (222);
(j) inserting the thread-like member (270) into the fifth hole from the curved outer surface (244) of the plate (222);
(k) inserting the thread-like member (270) into the fourth hole from the inner surface (242) of the plate (222);
(l) inserting the thread-like member (270) into the sixth hole from the curved outer surface (244) of the plate (222); and
(m) inserting the thread-like member (270) into the fifth hole from the inner surface (242) of the plate (222), and optionally
(n) repeating steps (h)-(m).

15. The method of claim 11, wherein after threading the thread-like member (270) through the plurality of holes (250), forming at least one knot with the thread-like member (270).

## Patentansprüche

1. Vorrichtung zum endovaskulären Ersetzen der Herzklappe eines Patienten, umfassend:
eine Befestigung (12, 212), die eine Außenfläche und eine Innenfläche aufweist, wobei die Befestigung (12, 212) von einer zusammengeklappten Zuführkonfiguration in eine vollständig entfaltete Konfiguration ausdehnbar ist; und
mehrere erste Verriegelungselemente (16, 216) und mehrere zweite Verriegelungselemente (18, 218), die an der Innenfläche der Befestigung (12, 212) befestigt sind, wobei jedes erste Verriegelungselement (16, 216) in ein zweites Verriegelungselement (18, 218) einrastbar ist, wobei eines der mehreren ersten Verriegelungselemente (16, 216) und der mehreren zweiten Verriegelungselemente (18, 218) gekrümmte Außenflächen (244, 264) aufweisen, wobei jede gekrümmte Außenfläche an der Innenfläche der Befestigung (12, 212) anstößt und jede Außenfläche zwischen einer ersten Seite und einer zweiten Seite gekrümmt ist,
**dadurch gekennzeichnet, dass** der Krümmungsradius der Außenfläche (244) zwischen dem Krümmungsradius der Befestigung (12, 212) in der zusammengeklappten Zuführkonfiguration und dem Krümmungsradius der Befestigung (12, 212) in der vollständig entfalteten Konfiguration liegt.

2. Vorrichtung nach Anspruch 1, wobei die Verriegelungselemente (16, 216; 18, 218) mit gekrümmten Außenflächen (244, 264) in mindestens der zusammengeklappten Zuführkonfiguration im Wesentlichen abstandsgleich beabstandet sind.

3. Vorrichtung nach Anspruch 2, wobei der Krümmungsradius der zweiten Verriegelungselemente (18, 218) durch den Krümmungsradius der Befestigung (12, 212) in der vollständig entfalteten Konfiguration, den Krümmungsradius der Befestigung (12, 212) in der zusammengeklappten Zuführkonfiguration und einen gewichteten Faktor bestimmt wird, wobei der gewichtete Faktor zwischen etwa 0,10 und 0,35 liegt.

4. Vorrichtung nach Anspruch 1, wobei die gekrümmte Außenfläche (244, 264) in der zusammengeklappten Zuführkonfiguration eine Bogenlänge zwischen etwa 3 % und etwa 30 % eines Umfangs der Innenfläche der Befestigung (12, 212) aufweist.

5. Vorrichtung nach Anspruch 1, wobei das zweite Verriegelungselement (18, 218) an der Befestigung (12, 212) mit einem Befestigungselement (270) befestigt ist.

6. Vorrichtung nach Anspruch 5, wobei das zweite Verriegelungselement (18, 218) mehrere erste Löcher in der Nähe eines ersten Endes und mehrere zweite Löcher in der Nähe eines zweiten Endes aufweist, wobei die mehreren ersten Löcher und die mehreren zweiten Löcher durch einen Teil der Außenfläche (244, 264) getrennt sind, wobei das Befestigungselement (270) durch die mehreren ersten Löcher und die mehreren zweiten Löcher gefädelt ist, um das zweite Verriegelungselement (18, 218) an der Befestigung (12, 212) zu befestigen, wobei insbesondere das Befestigungselement (270) die Außenfläche (244, 264) zwischen den mehreren ersten Löchern und den mehreren zweiten Löchern nur einmal überkreuzt.

7. Vorrichtung nach Anspruch 1, wobei die Befestigung (12, 212) von der zusammengeklappten Zuführkonfiguration zu einer Ruhezustandskonfiguration und von der Ruhezustandskonfiguration zur vollständig entfalteten Konfiguration ausdehnbar ist.

8. Vorrichtung nach Anspruch 1, wobei sowohl die mehreren ersten Verriegelungselemente (16, 216) als auch die mehreren zweiten Verriegelungselemente (18, 218) gekrümmte Außenflächen (244, 264) aufweisen.

9. Vorrichtung nach Anspruch 1, wobei das zweite Verriegelungselement (18, 218) Folgendes umfasst:
eine Platte (222) mit einer Innenfläche (242) und einer gekrümmten Außenfläche (244); und
einen Zahn, der drehbar in die Innenfläche (242) der Platte (222) eingerastet ist.

10. Vorrichtung nach Anspruch 9, wobei die Befestigung (12, 212) in der vollständig entfalteten Konfiguration einen Krümmungsradius aufweist, wobei die gekrümmte Außenfläche (244) des zweiten Verriegelungselements (18, 218) einen Krümmungsradius aufweist, wobei der Krümmungsradius der Befestigung (12, 212) im Wesentlichen gleich dem Krümmungsradius der gekrümmten Außenfläche (244) ist.

11. Verfahren zum Befestigen eines Verriegelungselements (18, 218) an einer Befestigung (12, 212) einer Vorrichtung zum endovaskulären Ersetzen der Herzklappe eines Patienten, wobei das Verfahren Folgendes umfasst:
Positionieren eines Verriegelungselements (18, 218) in Kontakt mit einer Innenfläche der Befestigung (12, 212), wobei die Befestigung (18, 218) eine Platte (222) mit einer gekrümmten Außenfläche (244), einer Innenfläche (242) und einer Dicke dazwischen umfasst, wobei mehrere Löcher (250) sich durch die Dicke der Platte (222) erstrecken, wobei die mehreren Löcher (250) mehrere erste Löcher und mehrere zweite Löcher umfassen, wobei die mehreren ersten Löcher und die mehreren zweiten Löcher durch einen Teil der gekrümmten Außenfläche (244) getrennt sind, wobei die gekrümmte Außenfläche (244) die Innenfläche der Befestigung (12, 212) kontaktiert; und
Fädeln eines fadenähnlichen Elements (270) durch die mehreren Löcher (250), derart, dass das fadenähnliche Element (270) die Außenfläche (244) zwischen den mehreren ersten Löchern und den mehreren zweiten Löchern nur einmal überkreuzt, um das Verriegelungselement (18, 218) sicher an der Befestigung (12, 212) zu befestigen,
**dadurch gekennzeichnet, dass** der Krümmungsradius der Außenfläche (244) zwischen dem Krümmungsradius der Befestigung (12, 212) in der zusammengeklappten Zuführkonfiguration und dem Krümmungsradius der Befestigung (12, 212) in der vollständig entfalteten Konfiguration liegt.

12. Verfahren nach Anspruch 11, wobei die mehreren ersten Löcher mindestens ein erstes Loch, ein zweites Loch und ein drittes Loch umfassen und die mehreren zweiten Löchern mindestens ein viertes Loch, ein fünftes Loch und ein sechstes Loch umfassen.

13. Verfahren nach Anspruch 12, wobei der Schritt des Fädelns des fadenähnlichen Elements (270) durch die mehreren Löcher Folgendes umfasst:
(a) Einführen eines fadenähnlichen Elements (270) in das erste Loch von der gekrümmten Außenfläche (244) der Platte (222) aus;
(b) Einführen des fadenähnlichen Elements (270) in das dritte Loch von der Innenfläche (242) der Platte (222) aus;
(c) Einführen des fadenähnlichen Elements (270) in das zweite Loch von der gekrümmten Außenfläche (244) der Platte (222) aus;
(d) Einführen des fadenähnlichen Elements (270) in das dritte Loch von der Innenfläche (242) der Platte (222) aus;
(e) Einführen des fadenähnlichen Elements (270) in das erste Loch von der gekrümmten Außenfläche (244) der Platte (222) aus; und
(f) Einführen des fadenähnlichen Elements (270) in das zweite Loch von der Innenfläche (242) der Platte (222) aus und wahlweise
(g) Wiederholen der Schritte (a) - (f).

14. Verfahren nach Anspruch 13, ferner Folgendes umfassend:
(h) Einführen des fadenähnlichen Elements (270) in das sechste Loch von der gekrümmten Außenfläche (244) der Platte (222) aus;
(i) Einführen des fadenähnlichen Elements (270) in das vierte Loch von der Innenfläche (242) der Platte (222) aus;
(j) Einführen des fadenähnlichen Elements (270) in das fünfte Loch von der gekrümmten Außenfläche (244) der Platte (222) aus;
(k) Einführen des fadenähnlichen Elements (270) in das vierte Loch von der Innenfläche (242) der Platte (222) aus;
(l) Einführen des fadenähnlichen Elements (270) in das sechste Loch von der gekrümmten Außenfläche (244) der Platte (222) aus; und
(m) Einführen des fadenähnlichen Elements (270) in das fünfte Loch von der Innenfläche (242) der Platte (222) aus und wahlweise
(n) Wiederholen der Schritte (h) - (m).

15. Verfahren nach Anspruch 11, wobei nach dem Fädeln des fadenähnlichen Elements (270) durch die mehreren Löcher (250) mindestens ein Knoten mit dem fadenähnlichen Element (270) gebildet wird.

## Revendications

1. Appareil pour le remplacement endovasculaire d'une valve cardiaque d'un patient, comprenant:
un ancrage (12, 212) présentant une surface extérieure et une surface intérieure, l'ancrage (12, 212) étant expansible à partir d'une configuration de pose comprimée jusqu'à une configuration complètement déployée; et
une pluralité de premiers éléments de verrouillage (16, 216) et une pluralité de seconds éléments de verrouillage (18, 218) attachés à la surface intérieure de l'ancrage (12, 212), chaque premier élément de verrouillage (16, 216) étant engageable avec un deuxième élément de verrouillage (18, 218), dans lequel un de la pluralité de premiers éléments de verrouillage (16, 216) et un de la pluralité de seconds éléments de verrouillage (18, 218) présentent des surfaces extérieures courbes (244, 264), dans lequel chaque surface extérieure courbe (244, 264) bute contre la surface intérieure de l'ancrage (12, 212) et chaque surface extérieure est incurvée entre un premier côté et un second côté,
**caractérisé en ce que** le rayon de courbure de la surface extérieure (244) est compris entre le rayon de courbure de l'ancrage (12, 212) dans la configuration de pose comprimée et le rayon de courbure de l'ancrage (12, 212) dans la configuration complètement déployée.

2. Appareil selon la revendication 1, dans lequel dans au moins la configuration de pose comprimée, les éléments de verrouillage (16, 216; 18, 218) présentant les surfaces extérieures courbes (244, 264) sont espacés de façon sensiblement équidistante.

3. Appareil selon la revendication 2, dans lequel le rayon de courbure des seconds éléments de verrouillage (18, 218) est déterminé par le rayon de courbure de l'ancrage (12, 212) dans la configuration complètement déployée, le rayon de courbure de l'ancrage (12, 212) dans la configuration de pose comprimée, et un facteur pondéré, en particulier dans lequel le facteur pondéré est compris entre 0,10 et 0,35.

4. Appareil selon la revendication 1, dans lequel dans la configuration de pose comprimée, la surface extérieure courbe (244, 264) présente une longueur d'arc qui est comprise entre environ 3 % et environ 30 % d'une circonférence de la surface intérieure de l'ancrage (12, 212).

5. Appareil selon la revendication 1, dans lequel le second élément de verrouillage (18, 218) est attaché à l'ancrage (12, 212) à l'aide d'un élément de fixation (270).

6. Appareil selon la revendication 5, dans lequel le second élément de verrouillage (18, 218) présente une première pluralité de trous à proximité d'une première extrémité et une seconde pluralité de trous à proximité d'une seconde extrémité, la première pluralité de trous et la seconde pluralité de trous étant séparées par une partie de la surface extérieure (244, 264), dans lequel l'élément de fixation (270) est enfilé à travers la première pluralité de trous et la seconde pluralité de trous afin d'attacher le second élément de verrouillage (18, 218) à l'ancrage (12, 212), en particulier dans lequel l'élément de fixation (270) croise la surface extérieure (244, 264) entre la première pluralité de trous et la deuxième pluralité de trous une seule fois.

7. Appareil selon la revendication 1, dans lequel l'ancrage (12, 212) est expansible à partir de la configuration de pose comprimée jusqu'à une configuration au repos et à partir de la configuration au repos jusqu'à la configuration complètement déployée.

8. Appareil selon la revendication 1, dans lequel la pluralité de premiers éléments de verrouillage (16, 216) et la pluralité de seconds éléments de verrouillage (18, 218) présentent toutes les deux des surfaces extérieures courbes (244, 264).

9. Appareil selon la revendication 1, dans lequel le second élément de verrouillage (18, 218) comprend:
une plaque (222) présentant une surface intérieure (242) et une surface extérieure courbe (244); et
une dent engagée de façon pivotante sur la surface intérieure (242) de la plaque (222).

10. Appareil selon la revendication 9, dans lequel dans la configuration complètement déployée, l'ancrage (12, 212) présente un rayon de courbure, dans lequel la surface extérieure courbe (244) du second élément de verrouillage (18, 218) présente un rayon de courbure, dans lequel le rayon de courbure de l'ancrage (12, 212) est sensiblement égal au rayon de courbure de la surface extérieure courbe (244).

11. Procédé de fixation d'un élément de verrouillage (18, 218) à un élément d'ancrage (12, 212) d'un appareil pour le remplacement endovasculaire d'une valve cardiaque d'un patient, le procédé comprenant les étapes suivantes:
placer un élément de verrouillage (18, 218) en contact avec une surface intérieure de l'élément d'ancrage (12, 212), dans lequel l'élément de verrouillage (18, 218) comprend une plate (222) présentant une surface extérieure courbe (244), une surface intérieure (242), et une épaisseur entre celles-ci présentant avec un pluralité de trous (250) qui s'étendent à travers l'épaisseur de la plaque (222), dans lequel la pluralité de trous (250) comprend une première pluralité de trous et une seconde pluralité de trous, dans lequel la première pluralité de trous et la seconde pluralité de trous sont séparées par une partie de la surface extérieure courbe (244); dans lequel la surface extérieure courbe (244) entre en contact avec la surface intérieure de l'élément d'ancrage (12, 212); et
enfiler un élément en forme de fil (270) à travers la pluralité de trous (250) de telle sorte que l'élément en forme de fil (270) croise une seule fois la surface extérieure (244) entre la première pluralité de trous et la seconde pluralité de trous afin d'attacher fermement l'élément de verrouillage (18, 218) à l'élément d'ancrage (12, 212),
**caractérisé en ce que** le rayon de courbure de la surface extérieure (244) est compris entre le rayon de courbure de l'ancrage (12, 212) dans la configuration de pose comprimée et le rayon de courbure de l'ancrage (12, 212) dans la configuration complètement déployée.

12. Procédé selon la revendication 11, dans lequel la première pluralité de trous comprend au moins un premier trou, un deuxième trou, et un troisième trou; et la seconde pluralité de trous comprend au moins un quatrième trou, un cinquième trou et un sixième trou.

13. Procédé selon la revendication 12, dans lequel l'étape d'enfilage de l'élément en forme de fil (270) à travers la pluralité de trous comprend les étapes suivantes:
(a) insérer un élément en forme de fil (270) dans le premier trou à partir de la surface extérieure courbe (244) de la plaque (222);
(b) insérer l'élément en forme de fil (270) dans le troisième trou à partir de la surface intérieure (242) de la plaque (222);
(c) insérer l'élément en forme de fil (270) dans le deuxième trou à partir de la surface extérieure courbe (244) de la plaque (222);
(d) insérer l'élément en forme de fil (270) dans le troisième trou à partir de la surface intérieure (242) de la plaque (222);
(e) insérer l'élément en forme de fil (270) dans le premier trou à partir de la surface extérieure courbe (244) de la plaque (222); et
(f) insérer l'élément en forme de fil (270) dans le deuxième trou à partir de la surface intérieure (242) de la plaque (222), et optionnellement
(g) répéter les étapes (a) à (f).

14. Procédé selon la revendication 13, comprenant en outre les étapes suivantes:
(h) insérer l'élément en forme de fil (270) dans le sixième trou à partir de la surface extérieure courbe (244) de la plaque (222);
(i) insérer l'élément en forme de fil (270) dans le quatrième trou à partir de la surface intérieure (242) de la plaque (222);
(j) insérer l'élément en forme de fil (270) dans le cinquième trou à partir de la surface extérieure courbe (244) de la plaque (222);
(k) insérer l'élément en forme de fil (270) dans le quatrième trou à partir de la surface intérieure (242) de la plaque (222);
(l) insérer l'élément en forme de fil (270) dans le sixième trou à partir de la surface extérieure courbe (244) de la plaque (222); et
(m) insérer l'élément en forme de fil (270) dans le cinquième trou à partir de la surface intérieure (242) de la plaque (222), et optionnellement
(n) répéter les étapes (h) à (m).

15. Procédé selon la revendication 11, dans lequel après l'enfilage de l'élément en forme de fil (270) à travers la pluralité de trous (250), on forme au moins un noeud avec l'élément en forme de fil (270).
